# EUROPÄISCHE PATENTSCHRIFT

(11) **EP 2 965 800 B1**
(45) Veröffentlichungstag und Bekanntmachung des Hinweises auf die Patenterteilung: **30.01.2019**
(21) Anmeldenummer: 14176209.6
(22) Anmeldetag: 08.07.2014
(51) Int. Cl.: B01D 53/04, B01D 53/047, B01D 53/22, C07C 1/12, C12M 1/00, C10L 3/10

(54) **Verfahren und Vorrichtung zu einer Gasaufbereitung**
Method and device for treating gas
Procédé et dispositif de traitement de gaz

(43) Veröffentlichungstag der Anmeldung: 13.01.2016
(73) Patentinhaber: Airbus Defence and Space GmbH, 82024 Taufkirchen (DE)
(72) Erfinder: Matthias Dr., Carsten, 88046 Friedrichshafen (DE); Jehle, Walter, 88263 Horgenzell (DE)
(74) Vertreter: Daub, Thomas

(56) Entgegenhaltungen:
- EP-A1- 2 438 975
- EP-A1- 2 468 384
- EP-A2- 2 532 729
- EP-A2- 2 682 450
- WO-A1-2011/097162
- DE-A1-102009 018 126
- DE-A1-102011 103 430
- US-A1- 2011 094 378
- US-A1- 2012 228 553

## Beschreibung

### Stand der Technik

Aus der EP 2 438 975 A1 sind bereits ein Verfahren und eine Vorrichtung bekannt, die einen Adsorber aus einem Adsorberharz aufweisen. In einer Adsorptionsphase wird Rohbiogas bei Umgebungsdruck durch den Adsorber geleitet. In einer Regenerationsphase wird der Adsorber mit einem Spülgas bei Temperaturen zwischen 20 - 100°C regeneriert.

Ferner sind aus der DE 10 2011 103 430 A1, der EP 2 682 450 A2 und der EP 2 532 729 A2 ebenfalls Verfahren zu einer Gasaufbereitung bekannt.

Eine Aufgabe der Erfindung besteht insbesondere darin, ein Verfahren mit einem hohen Wirkungsgrad bereitzustellen und/oder ein Verfahren, welches eine kompakte Vorrichtung ermöglicht. Die Aufgabe wird erfindungsgemäß durch die Merkmale des Patentanspruchs 1 gelöst, während vorteilhafte Ausgestaltungen und Weiterbildungen der Erfindung den Unteransprüchen und einem Nebenanspruch entnommen werden können.

### Vorteile der Erfindung

Es wird ein Verfahren zu einer Aufbereitung von Biogas einer Biogasanlage vorgeschlagen, bei dem in einem Verfahrensschritt zu einer CO₂-Abtrennung aus dem Biogas ein Membranprozess in einer Membraneinheit durchgeführt wird und in einem weiteren, nachgeschalteten Verfahrensschritt ein Adsorptions- und/oder Absorptionsprozess in Absorptionseinheiten, zur Gasreinigung zur Erzielung einer Methanreinheit von größer als 99,5 Vol.% durchgeführt wird, wobei der Adsorptions- und/oder Absorptionsprozess als Nachreinigung durchgeführt wird, wobei in dem Adsorptions- und/oder Absorptionsprozess ein Festkörperadsorber und/oder ein Festkörperabsorber genutzt wird, und zwar ein Festamin, wobei in einem weiteren Verfahrensschritt ein reaktiver Prozess, bei dem in einer Power-to-Gas Anlage in einem katalytisch aktivierten chemischen Prozess ein methanreiches Gasgemisch erzeugt wird, durchgeführt wird, wobei bei der Power-to-Gas Anlage elektrische Energie aus einer Anlage, wie insbesondere einer Windkraftanlage und/oder einer Solaranlage, verwendet wird, um mittels Elektrolyse Wasserstoff zu erzeugen, wobei in der Power-to-Gas Anlage erzeugter Wasserstoff während der Regeneration der Adsorptions- und/oder Absorptionseinheit zugeführt wird und wobei die Adsorptions- und/oder Absorptionseinheit eine Funktion einer CO₂-Speicherung und einer Gasvormischung für eine sich anschließende Methanisierung in der Power-to-Gas Anlage übernehmen, wobei der Wasserstoff während Regenerationsphasen der Absorptionseinheiten in die Absorptionseinheiten bei erhöhtem Druck eingeleitet wird und ein in den Absorptionseinheiten befindliches Absorptionsmaterial unter der H2-Atmosphäre thermisch regeneriert wird, wobei eine Abwärme der Power-to-Gas Anlage, und zwar von der Elektrolyse, die von einem Elektrolyseur durchgeführt wird, oder von einer Methanisierung, zur Regeneration des Absorptionsmaterials verwendet wird, wobei ein frei werdendes CO₂/H₂ Gasgemisch dann unter Druck einer sich anschließenden Methanisierung in der Power-to-Gas Anlage zugeführt wird. Darunter, dass der Adsorptions- und/oder Absorptionsprozess in einem "weiteren Verfahrensschritt" durchgeführt wird, soll in diesem Zusammenhang insbesondere verstanden werden, dass ein Adsorptions- und/oder Absorptionsmaterial getrennt, insbesondere beabstandet, insbesondere mit einem Abstand größer als 1 cm, von einer Membran angeordnet ist und/oder dass die Verfahrensschritte auch getrennt unabhängig voneinander betreibbar sind. Unter einem "reaktiven Prozess" soll in diesem Zusammenhang insbesondere ein Prozess verstanden werden, bei dem insbesondere in einem katalytisch aktivierten chemischen Prozess oder in einem biologischen Prozess, insbesondere mit einem Nährsubstrat, ein Gasgemisch erzeugt wird, wie insbesondere ein zumindest methanreiches Gasgemisch. Ganz besonders bevorzugt wird in dem reaktiven Prozess in einer Power-to-Gas Anlage zumindest ein methanreiches Gasgemisch aus einer CO₂-Quelle erzeugt. Dabei wird vorzugsweise Biogas als CO₂-Quelle genutzt. Unter einer "Power-to-Gas Anlage" soll dabei insbesondere eine Anlage verstanden werden, bei der elektrische Energie aus einer Anlage, wie insbesondere einer Windkraftanlage und/oder einer Solaranlage, verwendet wird, um mittels Elektrolyse Wasserstoff zu erzeugen. Dieser Wasserstoff wird dann insbesondere verwendet, um zusammen mit Kohlendioxid, wie insbesondere aus einer Biogasanlage oder aus einer anderen CO₂-Quelle, mittels eines Katalysators Methan zu erzeugen. Das Methan kann, insbesondere nach einer CO₂-Gasfeinreinigung mit der Adsorptions- und/oder Absorptionseinheit sowie einer Methantrocknung, in ein Erdgasnetz eingespeist werden. Die Adsorptions- und/oder Absorptionseinheit übernimmt neben der CO₂-Gasfeinreinigung vorzugsweise ebenfalls die Funktion der Methanresttrocknung auf sehr niedrige Taupunkttemperaturen im Produktgas Methan.

Es kann bei der Kombination der Verfahrensschritte eines Membranprozesses und eines Adorptions- und/oder Absorptionsprozesses eine Einsparung einer Membranstufe einer insbesondere mehrstufigen Membrananlage und damit eine erhebliche Membrananlagenverkleinerung erreicht werden, und zwar vorzugsweise um mehr als ein Drittel. Ferner kann eine besonders einfache Membranmodulverschaltung erreicht werden, und es können besonders hohe Gasqualitäten, insbesondere Bioerdgasqualitäten, mit einer Methanreinheit von größer als 99,5 Vol.%, erreicht werden, und zwar insbesondere, wenn der Adsorptions- und/oder Absorptionsprozess als Nachreinigung durchgeführt wird, wobei der Adsorptions- und/oder Absorptionsprozess insbesondere als Feinreinigung durchgeführt wird, bei der vorzugsweise eine Abreicherung von einer Kohlendioxidkonzentration im Prozessgas auf kleiner als 0,3 Vol.- % CO₂ durchgeführt wird. Der Adsorptions- und/oder Absorptionsprozess kann dabei verwendet werden, um das Retentat und/oder das Permeat eines Membranmoduls aufzubereiten, d.h. mit entsprechenden hohen oder niedrigen Drücken. Zudem ist eine Absenkung eines Feeddrucks der Membrananlage möglich und damit eine Reduzierung von Anlagenbetriebskosten. Ferner ist eine Verkleinerung eines Wärmetauschers der Membrananlage möglich, der für eine Feedgas-Kühlung benötigt wird. Unter "Bioerdgas" soll in diesem Zusammenhang insbesondere Methan verstanden werden, das nicht fossilen Ursprungs ist, sondern aus biogenen Stoffen erzeugt wurde.

Es kann bei der Kombination der Verfahrensschritte eines reaktiven Prozesses und eines Adsorptions- und/oder Absorptionsprozesses eine besonders hohe Gasqualität, insbesondere Bioerdgasqualitäten mit einer Methanreinheit von größer als 99,5 Vol.%, erreicht werden, und zwar insbesondere, wenn der reaktive Prozess als katalytischer Methanisierungsprozess ausgeführt wird und das im Bioerdgas enthaltene CO₂ mit H₂ aus einer Power-to-Gas Anlage katalytisch zu Methan und Wasser umgesetzt wird und anschließend ein Adsorptions- und/oder Absorptionsprozess als Nachreinigung durchgeführt wird, wobei der Adsorptions- und/oder Absorptionsprozess insbesondere als Feinreinigung durchgeführt wird, bei der vorzugsweise eine Abreicherung von einer Kohlendioxidkonzentration im Prozessgas auf kleiner als 0,3 Vol.-% CO₂ durchgeführt wird. Es können jedoch auch ein Verfahrensschritt mit einem Membranprozess, ein Verfahrensschritt mit einem reaktiven Prozess und ein Verfahrensschritt mit einem Adsorptions- und/oder Absorptionsprozess in verschiedenen, dem Fachmann als sinnvoll erscheinenden Varianten kombiniert werden.

Grundsätzlich sind verschiedene, dem Fachmann als sinnvoll erscheinende Adsorber- und/oder Absorbermaterialien denkbar, wie insbesondere flüssige oder gasförmige Adsorber- und/oder Absorbermaterialien. Besonders vorteilhaft wird jedoch zumindest ein Festkörperadsorber und/oder ein Festkörperabsorber genutzt, und zwar ganz besonders bevorzugt ein Absorber- und/oder Adsorberharz. Besonders eignet sich ein Amin, und zwar bevorzugt ein Festamin. Das Absorptions- und/oder Adsorptionsmaterial weist dabei vorzugsweise eine Selektivität bei Standardbedingungen, d.h. bei einer Temperatur von 25°C und einem Druck von 1 bar_{abs}, größer als 100, besonders bevorzugt größer als 150 und ganz bevorzugt größer als 200 auf. Unter einer "Selektivität" soll dabei ein Beladungsverhältnis, insbesondere ein Festamin-Beladungsverhältnis verstanden werden, insbesondere ein Beladungsverhältnis zwischen CO₂ und CH₄. Das Absorptions- und/oder Adsorptionsmaterial wird vorzugsweise in Form einer Schüttung verwendet. Unter einer "Schüttung" soll in diesem Zusammenhang insbesondere verstanden werden, dass das Material in einem körnigen und/oder auch stückigen Gemenge vorliegt, das in einer schüttfähigen Form vorliegt. Mit einem Festamin können vorteilhaft niedrige Regenerationstemperaturen erreicht werden, wodurch besonders vorteilhaft Abwärme von anderen Einheiten, wie insbesondere eines Blockheizkraftwerks (BHKWs), einer Membraneinheit und/oder einer Power-to-Gas Anlage genutzt werden kann. Ferner können eine hohe Lebensdauer und eine besonders hohe Gasreinheit erzielt werden. Alternativ zu einem Amin sind jedoch auch grundsätzlich Molekularsiebe, beispielsweise aus Aktivkohle usw., denkbar.

In einer weiteren Ausgestaltung der Erfindung wird vorgeschlagen, dass zumindest eine Adsorptions- und/oder Absorptionseinheit in zumindest einem Verfahrensschritt gekühlt oder erwärmt wird, wodurch der Adsorptions- und/oder Absorptionsprozess und/oder ein Regenerationsprozess besonders vorteilhaft unterstützt werden kann. Unter "gekühlt" oder "erwärmt" soll dabei insbesondere verstanden werden, dass aktiv, insbesondere mit einer dritten Einheit, Wärme abgeführt oder Wärme zugeführt wird. Vorzugsweise wird eine sogenannte Temperatur Swing Adsorption- und/oder Absorption durchgeführt, bei der bei einer niedrigen Temperatur eine Adsorption und/oder Absorption durchgeführt wird und bei einer höheren Temperatur eine Regeneration durchgeführt wird. Die Adsorption und/oder Absorption findet dabei vorteilhaft bei einer Temperatur kleiner als 40°C, vorzugsweise kleiner als 30°C und ganz besonders bevorzugt kleiner als 20°C statt, während die Regeneration vorteilhaft bei einer Temperatur größer als 70°, vorzugsweise bei einer Temperatur größer als 80°C und ganz besonders bevorzugt größer als 90°C durchgeführt wird. Vorzugsweise wird die Regeneration jedoch bei einer Temperatur kleiner als 120°C durchgeführt.

Vorzugsweise wird zu Beginn der Regeneration der Adsorptions- und/oder Absorptionseinheit eine Methanrückgewinnung mittels einer Vakuumpumpe realisiert.

Wird Wärmeenergie von zumindest einer Adsorptions- und/oder Absorptionseinheit zu zumindest einer weiteren Adsorptions- und/oder Absorptionseinheit übertragen und/oder wird Druckenergie von zumindest einer Adsorptions- und/oder Absorptionseinheit zu zumindest einer weiteren Adsorptions- und/oder Absorptionseinheit übertragen, kann durch eine vorteilhafte Wärmeintegration bzw. Druckintegration Energie eingespart und ein hoher Wirkungsgrad erreicht werden. Vorzugsweise sind dabei mehrere Adsorptions- und/oder Absorptionseinheiten miteinander verschaltet. Vorzugsweise werden die Adsorptions- und/oder Absorptionseinheiten im Wechsel gegenseitig bedruckt oder evakuiert. Es wird vorteilhaft eine sogenannte Pressure Swing Adsorption- und/oder Absorption durchgeführt, bei der bei einem höheren Druck eine Adsorption und/oder Absorption durchgeführt wird und bei einem niedrigeren Druck eine Regeneration durchgeführt wird.

Ferner wird vorgeschlagen, dass zu einer Temperierung, d.h. Kühlung und/oder Erwärmung, einer Adsorptions- und/oder Absorptionseinheit Abwärme genutzt wird, und zwar insbesondere Abwärme eines Kraftwerks, insbesondere eines Blockheizkraftwerks, und/oder Abwärme des Membranprozesses und/oder ganz besonders bevorzugt von einer Power-to-Gas Anlage, wodurch wiederum Energie eingespart werden kann. Bei einer Power-to-Gas Anlage kann die Abwärme bevorzugt eines Elektrolyseurs und/oder freiwerdende Reaktionswärme der katalytischen Methanisierung zu einer Regeneration eines Adsorber- und Absorbermaterials genutzt werden. Insbesondere kann zu einer Kühlung im Adsorptions- und/oder Absorptionsprozess eine Kälteanlage verwendet werden, welche Abwärme zur Kälteerzeugung verwendet. Besonders vorteilhaft sind mehrere Adsorptions- und/oder Absorptionseinheiten miteinander verschaltet und eine Wärmeintegration der Adsorptions- und/oder Absorptionseinheiten wird mittels eines umgewälzten Wärmeträgerfluids untereinander und/oder im Verbund mit einem Blockheizkraftwerk und/oder mit einer Membraneinheit und/oder mit der Power-to-Gas Anlage realisiert. Eine anfallende Adsorptions- und/oder Absorptionswärme während der CO₂-Adsorption und/oder Absorption kann vorteilhaft der Biogasanlage zugeführt werden. Bei der Methanerzeugung (Methanisierung) in einer Power-to-Gas Anlage wird durch die exotherme Reaktion CO₂+4H₂ ↔ CH₄ + 2H₂O Wärme frei, die vorzugsweise in der Biogasanlage und/oder vorteilhaft in einem entsprechenden erfindungsgemäßen Verfahren, insbesondere zur Regeneration der Adsorptions- und/oder Absorptionseinheit genutzt werden kann.

Erfindungsgemäß wird vorgeschlagen, dass zumindest einer Adsorptions- und/oder Absorptionseinheit Wasserstoff zugeführt wird, und zwar insbesondere von einer Power-to-Gas Anlage. Der Wasserstoff wird dabei vorzugsweise während einer Regeneration in die Adsorptions- und/oder Absorptionseinheit eingeleitet, wodurch die Regeneration besonders effizient durchgeführt werden kann, und zwar insbesondere aufgrund einer erzielbaren verbesserten Wärmeleitfähigkeit eines Adsorptions-und/oder Absorptionsmaterials unter einer H₂-Atmosphäre, und zwar vorzugsweise aufgrund einer verbesserten Wärmeleitfähigkeit einer Adsorptions- und/oder Absorptionsharzschüttung. Die Regeneration findet vorzugsweise bei erhöhtem
Druck, d.h. insbesondere bei einem Druck größer als 2 bar_{abs}. und besonders vorteilhaft größer als 4 bar_{abs}. und unter einer H2-Atmosphäre statt. Die Edukte H₂ und CO₂ werden vorzugsweise bei einem so hohen Druckniveau am Ende einer Desorption an die Power-to-Gas Anlage abgegeben, dass in einer anschließenden Methanisierung das Produktgas Methan ohne Nachverdichtung direkt ins Erdgasnetz eingespeist werden kann. Hierdurch kann energetisch besonders günstig eine Druckerhöhung vor einer Elektrolyse innerhalb der Power-to-Gas Anlage erreicht werden. Eine Regeneration ohne Wasserstoffzufuhr in die Adsorptions- und/oder Absorptionseinheit findet vorzugsweise bei einem Druck kleiner als 1,5 bar_{abs}. statt.

Die Adsorptions- und/oder Absorptionseinheit und insbesondere eine darin befindliche Adsorptions- und/oder Absorptionsharzschüttung kann vorteilhaft eine Funktion einer CO₂-Speicherung und einer Gasvormischung für eine sich anschließende Methanisierung in der Power-to-Gas Anlage übernehmen. Ferner können die Edukte CO₂ und H₂ für die Methanisierung bei erhöhtem Druck gewonnen werden, was sich günstig auf eine Gleichgewichtslage und einen erzielbaren Umsatz der Methanisierung auswirkt. Die Regeneration der Adsorptions- und/oder Absorptionseinheit und insbesondere die Regeneration einer Adsorptions- und/oder Absorptionsharzschüttung unter einer Wasserstoffatmosphäre, wird über die Wasserstoffzufuhr sowie einen Regenerationsdruck und eine Temperatur vorteilhaft so geregelt, dass die Edukte CO₂ und H₂ für eine sich anschließende Methanisierung in der Power-to-Gas Anlage zumindest im Wesentlichen im stöchiometrischen Verhältnis aus der Adsorptions- und/oder Absorptionseinheit abgeführt werden können. Unter zumindest im Wesentlichen soll dabei insbesondere verstanden werden, dass eine Abweichung kleiner als 20% und besonders bevorzugt kleiner als 10% vorliegt.

Ferner wird in Anspruch 5 eine Vorrichtung zu einer Durchführung eines erfindungsgemäßen Verfahrens vorgeschlagen.

Vorzugsweise umfasst die Vorrichtung zumindest eine Heiz- und/oder Kühleinheit, wodurch, wie oben beschrieben, vorteilhaft eine aktive Erwärmung und/oder aktive Kühlung durchgeführt werden kann.

Umfasst die Heiz- und/oder Kühleinheit zumindest einen Wärmetauscher, kann eine besonders effektive Wärmeübertragung erreicht werden, und zwar insbesondere, wenn in den Wärmetauscher wenigstens ein Adsorptions- und/oder Absorptionselement integriert ist. Dabei soll unter "integriert" insbesondere verstanden werden, dass Teile des Wärmetauschers mit einem Adsorptions- und/oder Absorptionsmaterial befüllt und/oder beschichtet sind. Besonders vorteilhaft umfasst der Wärmetauscher wenigstens ein Rohr, welches zumindest teilweise mit einem Adsorptions- und/oder Absorptionsmaterial gefüllt ist, und zwar besonders vorteilhaft mit einem Harz und ganz besonders bevorzugt mit einer Harzschüttung, insbesondere einer Festamin-Harzschüttung. Das Biogas wird dabei vorzugsweise durch das Adsorptions- und/oder Absorptionsmaterial, insbesondere durch die Harzschüttung in dem Rohr, geleitet. In einem Mantelraum des Wärmetauschers, der vorzugsweise als Rohrbündelwärmetauscher ausgebildet sein kann, wird dann vorzugsweise ein Wärmeträgerfluid geführt.

In einer weiteren Ausgestaltung wird vorgeschlagen, dass das wenigstens eine Rohr zumindest eine erste und eine zweite Wandung aufweist und das Adsorptions- und/oder Absorptionsmaterial zumindest teilweise zwischen den Wandungen angeordnet ist, wodurch das Adsorptions- und/oder Absorptionsmaterial vorteilhaft von innen und von außen beheizbar ist. Alternativ kann das Rohr jedoch auch nur eine Wandung aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine vereinfachte Prozessdarstellung mit einer Membraneinheit,
- Fig. 2: einen schematisch dargestellten Ausschnitt einer erfindungsgemäßen Vorrichtung mit einer Absorptionseinheit,
- Fig. 3: einen schematisch dargestellten Ausschnitt einer Schnittdarstellung durch die Absorptionseinheit aus Fig. 2,
- Fig. 4: einen schematisch dargestellten Ausschnitt einer Schnittdarstellung einer zu der in Figur 2 dargestellten alternativen Absorptionseinheit,
- Fig. 5: vereinfacht dargestellte Prozessabläufe bei mehreren verschalteten Absorptionseinheiten und
- Fig. 6: eine vereinfachte Darstellung eines Fließbilds der Vorrichtung,
- Fig. 7: eine nicht erfindungsgemäße weitere vereinfachte Prozessdarstellung mit einer Membraneinheit und einer Power-to-Gas Anlage und
- Fig. 8: eine nicht erfindungsgemäße weiter vereinfachte Prozessdarstellung mit einer Power-to-Gas Anlage und ohne Membraneinheit.

### Beschreibung der Ausführungsbeispiele

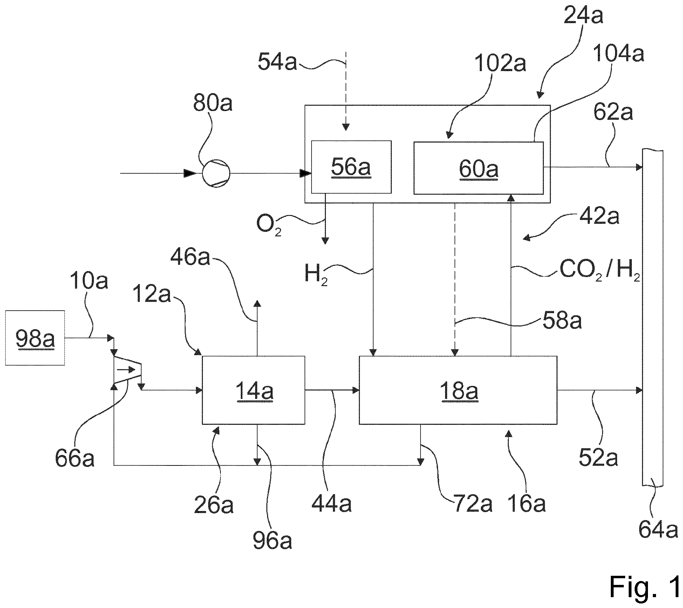

Figur 1 zeigt eine vereinfachte Prozessdarstellung bzw. eine vereinfachte Darstellung eines erfindungsgemäßen Verfahrens zu einer Gasaufbereitung, und zwar zu einer Aufbereitung von Biogas 10a in einer Biogasanlage 98a, bei dem in einem Verfahrensschritt 12a zu einer CO₂-Abtrennung aus dem Biogas 10a ein Membranprozess 14a in einer Membraneinheit 26a durchgeführt wird und in einem weiteren, nachgeschalteten Verfahrensschritt 16a ein Absorptionsprozess 18a in Absorptionseinheiten 22a, 22a', 22a", 22a'" durchgeführt wird (vgl. Figuren 1, 2 und 6). In der Membraneinheit 26a werden nicht näher dargestellte Hohlfasermembrane eingesetzt, wobei grundsätzlich auch andere, dem Fachmann als sinnvoll erscheinende Membrane denkbar sind. Das Biogas 10a wird mittels eines Kompressors 66a der Membraneinheit 26a zugeführt, und zwar wird das Biogas 10a durch die Hohlfasermembrane gepumpt. Der Membranprozess 14a, welcher mittels einer Rückführung 96a mehrstufig ausgebildet ist, wird als Vorreinigung, insbesondere zu einer Abreicherung einer CO₂-Konzentration im Biogas 10a von ca. 45 Vol.-% auf 3 - 5 Vol.-% CO₂, vor dem Absorptionsprozess 18a durchgeführt. Der Absorptionsprozess 18a übernimmt im Anschluss eine Nachreinigung, und zwar insbesondere eine Feinreinigung, mit vorzugsweise einer Anreicherung eines Bioerdgases 52a auf eine Methankonzentration größer als 99%. Ein Retentat 44a der Membraneinheit 26a wird mittels des Kompressors 66a den Absorptionseinheiten 22a, 22a, 22a", 22a'" zur Feinreinigung zugeführt. Grundsätzlich könnten jedoch auch die Absorptionseinheiten 22a, 22a', 22a", 22a'" zusätzlich oder alternativ zu einer Aufbereitung eines Permeats 46a der Membraneinheit 26a eingesetzt werden. Das Permeat 46a besteht dabei nahezu vollständig aus CO₂ und einem Anteil CH₄ bzw. Methan kleiner 0,5 Vol.-%. Ein erfindungsgemäßer Gesamtprozess nutzt vorteilhaft unterschiedliche Selektivitäten der Membraneinheit 26a und der Absorptionseinheiten 22a, 22a', 22a", 22a'" zu einer CO₂/CH₄-Gastrennung aus.

Das Verfahren kann grundsätzlich allein gemeinsam mit einer Biogasanlage 98a betrieben werden oder kann vorteilhaft bei einer Kombination einer Biogasanlage 98a mit weiteren, dem Fachmann als sinnvoll erscheinenden Anlagen betrieben werden, wie insbesondere besonders vorteilhaft in Kombination mit einer Power-to-Gas Anlage 24a. Hierzu weist die erfindungsgemäße Vorrichtung einen Anlagenanschluss 42a zum Anschluss einer reaktiven Anlage, und zwar zum Anschluss der Power-to-Gas Anlage 24a auf. Die Power-to-Gas Anlage 24a, der Wasser mittels einer Wasserpumpe 80a zugeführt wird, nutzt Strom 54a, wie vorzugsweise aus Anlagen, die aus erneuerbaren Energiequellen Strom gewinnen, wie insbesondere Windkraftanlagen, Solaranlagen usw., um damit mittels einer Elektrolyse 56a H₂ und O₂ bei erhöhtem Druck, vorzugsweise bei einem Druck höher als 5 bar_{abs}, insbesondere bei einem Druck von zumindest im Wesentlichen 10 bar_{abs}. zu erzeugen. Unter zumindest im Wesentlichen soll dabei insbesondere verstanden werden, dass eine Abweichung kleiner als 20% und besonders bevorzugt kleiner als 10% vorliegt.

Die Power-to-Gas Anlage 24a wird vorzugsweise mit einer konstanten Strommenge versorgt, um eine hohe Lebensdauer zu erzielen. Die Power-to-Gas Anlage 24a kann hierfür mit einer oder mehreren Anlagen gekoppelt sein. Grundsätzlich ist auch denkbar, dass bei der Power-to-Gas Anlage 24a Überschussenergie einer Anlage genutzt wird. In der Power-to-Gas Anlage 24a wird in einem Verfahrensschritt 102a in einem reaktiven Prozess 104a Methan 62a erzeugt.

Der Wasserstoff wird während Regenerationsphasen der Absorptionseinheiten 22a, 22a', 22a", 22a'" in die Absorptionseinheiten 22a, 22a', 22a", 22a'" bei erhöhtem Druck eingeleitet und ein in den Absorptionseinheiten 22a, 22a', 22a", 22a'" befindliches Absorptionsmaterial wird unter H₂-Atmosphäre thermisch regeneriert. Eine Abwärme 58a der Power-to-Gas Anlage 24a, und zwar von der Elektrolyse 56a, die von einem Elektrolyseur durchgeführt wird, und von einer Methanisierung 60a, wird zur Regeneration des Absorptionsmaterials verwendet. Ein frei werdendes CO₂/H₂ Gasgemisch wird dann unter Druck, vorzugsweise zumindest im Wesentlichen bei 10 bar_{abs}. und nahe bzw. zumindest im Wesentlichen bei einem stöchiometrischen Verhältnis ¼, einer sich anschließenden Methanisierung 60a in der Power-to-Gas Anlage 24a zugeführt. Die Methanisierung 60a erfolgt bevorzugt bei einem Druckniveau, sodass keine Nachverdichtung des sich aus der Methanisierung 60a ergebenden Methans 62a zur Einspeisung in ein Erdgasnetz 64a benötigt wird. Vor der Einspeisung in das Erdgasnetz 64a wird das Methan 62a getrocknet.

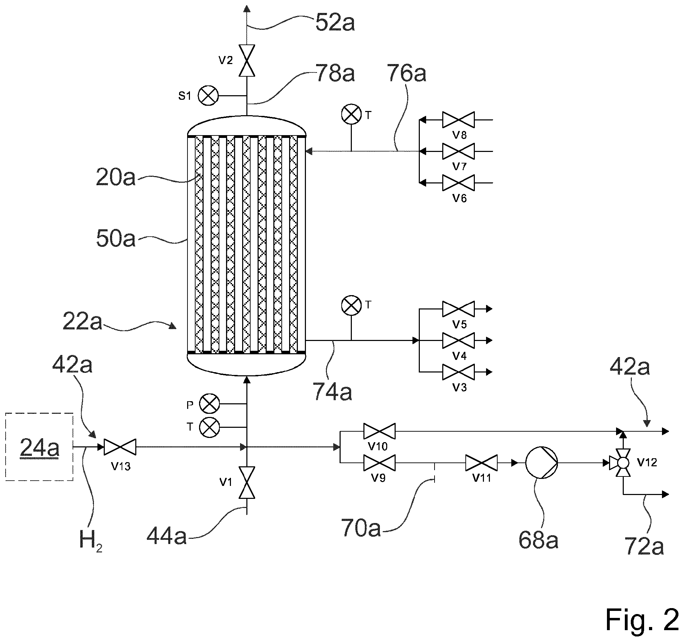

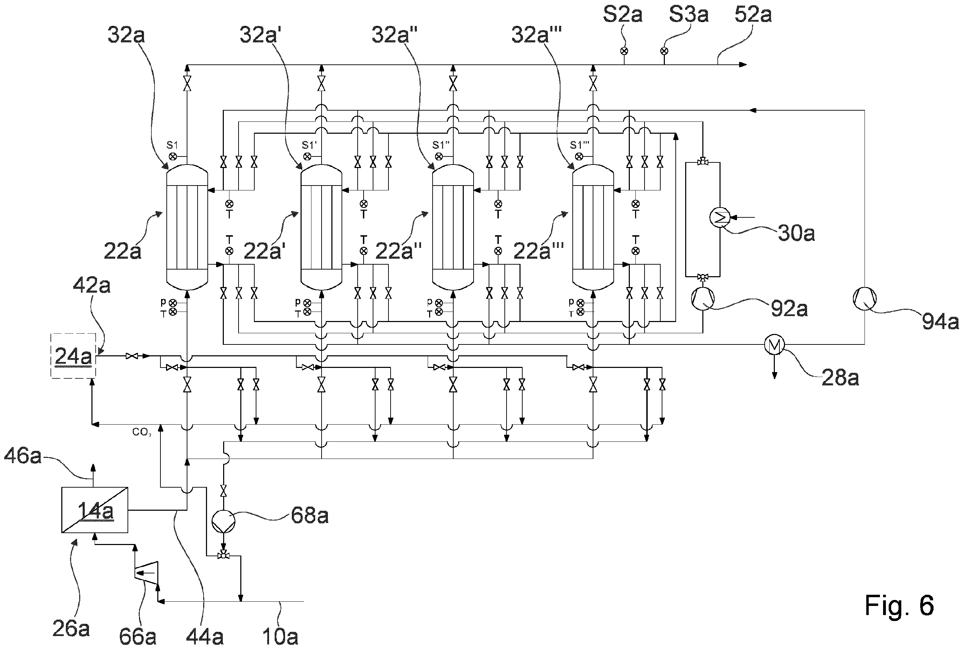

Die Figur 2 zeigt einen schematisch dargestellten Ausschnitt einer erfindungsgemäßen Vorrichtung zur Durchführung des Verfahrens. Die Vorrichtung umfasst die Membraneinheit 26a und die Absorptionseinheiten 22a, 22a', 22a", 22a'" sowie Heiz- und Kühleinheiten 28a, 30a, 32a (Figuren 2 und 6).

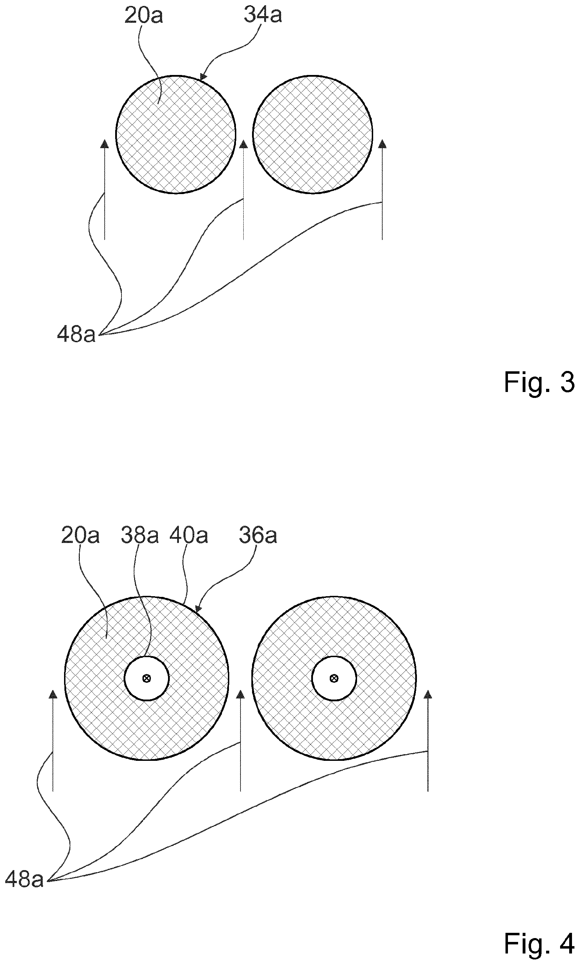

Im Absorptionsprozess 18a wird ein Festkörperabsorber 20a genutzt, und zwar ein Festamin. Das Festamin ist in heiz- und kühlbare Apparate integriert, und zwar in Wärmetauscher der Heiz- und Kühleinheiten 32a, 32a', 32a", 32a"', somit sind Absorptionselemente in den Wärmetauschern integriert (Figuren 2, 3, 4 und 6). Die Absorptionseinheiten 22a, 22a', 22a", 22a'" weisen dabei eine Bauform auf, die vergleichbar ist, mit der eines Rohrbündelwärmetauschers. Das Festamin ist in Rohre 34a in Form einer Schüttung eingefüllt (Figuren 2, 3 und 4). Ein Wärmeträgerfluid 48a wird mantelseitig durch die Wärmetauscher geleitet. Die Rohre 34a sind druckstabil ausgeführt und bestehen aus Metall, vorzugsweise aus Edelstahl. Ein äußeres Gehäuse 50a der Wärmetauscher bzw. der Absorptionseinheiten 22a, 22a', 22a", 22a'" muss nicht druckstabil sein und ist aus einem Kunststoffmaterial gefertigt. Grundsätzlich sind jedoch sowohl für die Rohre 34a als auch für das Gehäuse 50a andere, dem Fachmann als sinnvoll erscheinende Materialien denkbar. Alternativ zu den Rohren 34a, die einwandig ausgebildet sind, könnten die Absorptionseinheiten 22a, 22a', 22a", 22a'" auch Rohre 36a aufweisen, die eine erste und eine zweite Wandung 38a, 40a aufweisen, wobei das Festamin in einer Ringspalte zwischen den Wandungen 38a, 40a angeordnet ist (Fig. 4). Die Rohre 34a werden im Betrieb mantelseitig von dem Wärmeträgerfluid 48a umströmt, während die Rohre 36a im Betrieb mantelseitig und im Inneren von dem Wärmeträgerfluid 48a umströmt werden. Ein Design mit den Rohren 36a verspricht eine besonders effiziente und schnelle Regeneration der Absorptionseinheiten 22a, 22a', 22a", 22a'" bzw. eines Absorptionsharzes. Ein Design mit den Rohren 34a kann besonders einfach gefertigt werden.

Prozessgas bzw. das Retentat 44a aus der Membraneinheit 26a wird in den Rohren 34a durch das Festamin, das in Form einer Schüttung vorliegt, geleitet. Dabei wird das CO₂ hochselektiv am Festamin während des Absorptionsprozesses 18a gebunden und Bioerdgas 52a kann an Ausgängen 78a der Absorptionseinheiten 22a, 22a', 22a", 22a'" gewonnen und in das Erdgasnetz 64a eingespeist werden.

Die Absorptionseinheiten 22a, 22a', 22a", 22a'" weisen jeweils mehrere Ventile V1, V2, V9 - V13 auf einer Gasseite sowie mehrere Ventile V3 - V8 auf einer Wärmeträgerfluidseite auf (Fig. 2). An den Ventilen V1, V2, V9 - V13 und V3 - V8 sind jeweils Rohranschlüsse vorgesehen.

Die Ventile V1, V2, V9, V10, V11, V13 auf der Gasseite werden benötigt, um einen PSA (Pressure Swing Absorption) Prozess realisieren zu können. Die Absorption des CO₂ am Festamin erfolgt dabei bei einem hohen Druck, vorzugsweise bei einem Druck zwischen 4 bar_{abs}. und 20 bar_{abs}. Die Regeneration erfolgt bei einem Gesamtprozess ohne Power-to-Gas Anlage 24a bei einem niedrigen Druck, und zwar vorzugsweise bei einem Druck zwischen 0,1 bar_{abs}. und 1,5 bar_{abs}. Bei einem Gesamtprozess mit der Power-to-Gas Anlage 24a erfolgt die Regeneration bei einem erhöhten Druck, und zwar vorzugsweise bei einem Druck zwischen 4 bar_{abs}. und 20 bar_{abs}. Es wird dabei H₂ während der Regeneration in die Rohre 34a der Absorptionseinheiten 22a, 22a', 22a", 22a'" mittels eines Ventils V13 eingeleitet und die Regeneration des Festamins wird unter Wasserstoffatmosphäre bei erhöhtem Druck durchgeführt. Die Wasserstoffzufuhr sowie die Regenerationstemperatur in den Rohren 34a wird dabei so geregelt, dass sich ein molares Mischungsverhältnis in der Gasphase von R_{H2/CO2} = 4 +/- 20 % einstellt.

Die Ventile V9 - V12 werden dazu benötigt, eine Methanrückgewinnung über einen Anschluss 72a mit einer Vakuumpumpe 68a am Ende einer Absorptionsphase und vor Beginn der Regeneration des Festamins zu realisieren und Methan 62a aus einer freien Gasphase der Harzschüttung abzusaugen und dieses zum Kompressor 66a zurückzuführen oder der Power-to-Gas Anlage 24a über den Power-to-Gas Anlagenanschluss 42a zuzuführen (Figuren 1 und 2). Die Ventile V9 - V12 werden auch dazu benötigt, CO₂ aus den Absorptionseinheiten 22a, 22a', 22a", 22a'" während Regenerationsphasen abzusaugen, und zwar im Falle eines Betriebs ohne Power-to-Gas Anlage 24a. Ferner werden die Ventile V9 - V12 dazu benötigt, Absorptionseinheiten 22a, 22a', 22a", 22a'" beim PSA Prozess miteinander zu verschalten und die Absorptionseinheiten 22a, 22a', 22a", 22a'" im Wechsel zu befüllen oder zu entleeren und damit Betriebszeiten der Vakuumpumpe 68a und damit verbundene Betriebskosten zu minimieren. Zwischen den Ventilen V9 und V11 ist ein Anschluss 70a zu einer weiteren Absorptionseinheit 22a', 22a", 22a'" vorgesehen.

Die Ventile V3 - V8 auf der Wärmeträgerfluidseite werden benötigt, um einen TSA (Temperature Swing Absorption) Prozess realisieren zu können. Die Ventile V3 - V5 sind dabei mit einem Wärmeträgerfluidausgang 74a und die Ventile V6 - V8 sind mit einem Wärmeträgerfluideingang 76a verschaltet. Die Absorption des CO₂ am Festamin erfolgt dabei bei niedriger Temperatur und die Regeneration bei hoher Temperatur (max. 110°C). Dabei wird eine anfallende Absorptionswärme 82a während der CO₂-Absorption am Festamin über den Wärmeträgerfluidausgang 74a der Biogasanlage 98a zugeführt, wodurch die Absorptionseinheiten 22a 22a', 22a", 22a'" bzw. Festaminschüttungen gekühlt werden. Ferner sind die Absorptionseinheiten 22a, 22a', 22a", 22a'" über die Ventile V4 - V7 miteinander verschaltet, um eine Wärmeintegration der Absorptionseinheiten 22a, 22a', 22a", 22a'" untereinander sowie gegebenenfalls mit einem Blockheizkraftwerk, der Membraneinheit 26a und/oder Power-to-Gas Anlage 24a zu realisieren. Ferner können die Ventile V3 - V8 zur Aufheizung und zur Kühlung der Absorptionseinheiten 22a, 22a', 22a", 22a'" während der Regeneration des Festamins genutzt werden.

Die Absorptionseinheiten 22a, 22a', 22a", 22a'" weisen jeweils an ihrem Ausgang 78a einen CO₂-Sensor S1, S1', S1", S1'" auf, mit dem eine CO₂-Konzentration im Bioerdgas 52a gemessen und über den die Prozessabläufe Absorption und Regeneration der Absorptionseinheiten 22a, 22a', 22a", 22a'" zeitlich geregelt werden.

Temperatursensoren T und Drucksensoren P an den Absorptionseinheiten 22a, 22a', 22a", 22a'" dienen zusätzlich zu einer zeitlichen Regelung der Prozessschritte während Absorptions- und Regenerationsphasen.

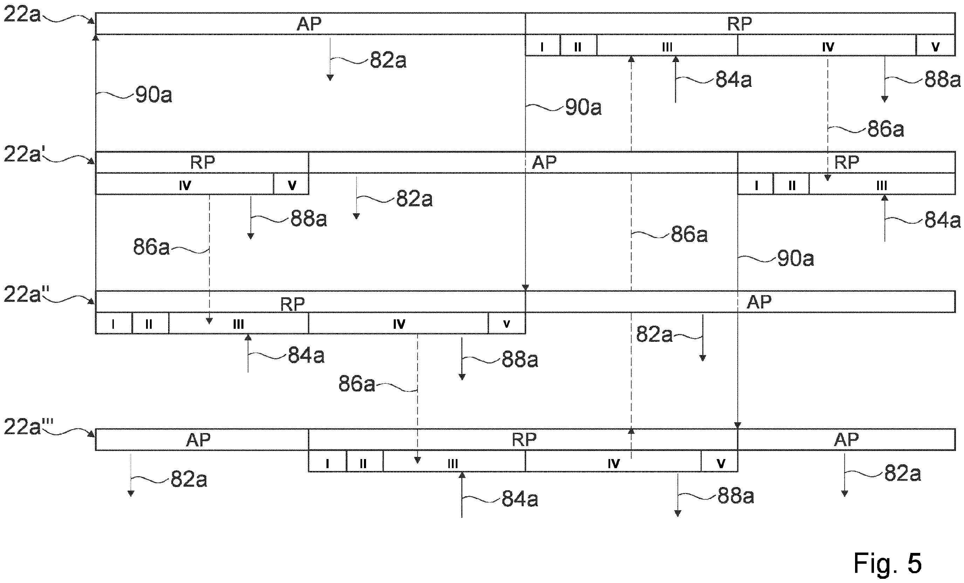

Durch die mehreren Absorptionseinheiten 22a, 22a', 22a", 22a'" und eine Verschaltung derselben untereinander, kann ein quasikontinuierlicher Prozess erreicht werden, bei dem die Absorptionseinheiten 22a, 22a', 22a", 22a'" Absorptions- und Regenerationsphasen zeitversetzt durchlaufen (Fig. 5).

Der Absorptionsprozess 18a mit dem CO₂-Absorberharz umfasst eine Absorptionsphase AP als Feinreinigungsstufe, in der das CO₂ am Absorptionsmaterial, und zwar am Festamin, bei einem Druck zwischen 1 bar_{abs}. und 15 bar_{abs}. gebunden und Absorptionswärme 82a gewonnen wird. Die Absorptionswärme 82a wird aus den Absorptionseinheiten 22a, 22a', 22a", 22a'" mittels des Wärmeträgerfluids 48a abgeführt, wodurch das Absorptionsmaterial mittels des Wärmeträgerfluids 48a gekühlt wird. Die Absorptionswärme 82a wird der Biogasanlage 98a, insbesondere einem Fermenter, zugeführt. Das Bioerdgas 52a kann während dieser Phase am Ausgang der Vorrichtung gewonnen und in das Erdgasnetz 64a eingespeist werden.

Ferner umfasst der Absorptionsprozess 18a eine Regenerationsphase RP, in der das CO₂ aus dem Absorptionsmaterial, und zwar aus dem Festamin, ausgetrieben wird, wobei sich die Regenerationsphase RP in weitere Phasen I - V aufgeteilt:
I. Druckabsenkung/Evakuierung des Absorptionsmaterials und Methanrückgewinnung aus einem Leerraum der Absorptionseinheit 22a, 22a', 22a", 22a'".
   Die Evakuierung findet dabei vorzugsweise isotherm statt.
II. Wasserstoffzufuhr aus der Power-to-Gas Anlage 24a (bevorzugt jedoch optional)
III. Aufheizphase des Absorptionsmaterials mittels des Wärmeträgerfluids 48a, vorzugsweise auf eine Temperatur kleiner als 120°C. Hierbei kann Abwärme 84a eines BHKWs und/oder der Membraneinheit 26a und/oder der Power-to-Gas Anlage 24a und/oder einer der anderen Absorptionseinheiten 22a, 22a', 22a", 22a'" genutzt werden. Hierzu ist eine Heiz- und Kühleinheit 30a in Form eines Wärmetauschers vorgesehen (Fig. 6). Eine CO₂-Abfuhr findet dabei entweder unter Verwendung der Vakuumpumpe 68a (Prozess ohne Power-to-Gas Anlage 24a) und/oder unter Verwendung von H₂ als Spülgas (Prozess mit Power-to-Gas Anlage 24a und direkter H₂-Zufuhr) statt. Dabei wird das Spülgas vorzugsweise unter erhöhtem Druck zugeführt. In diesem Fall kann zumindest zeitweise auf einen Einsatz der Vakuumpumpe 68a oder auch vollständig auf die Vakuumpumpe 68a verzichtet werden. Für eine Zirkulation des Wärmeträgerfluids 48a sind zwei Pumpen 92a, 94a vorgesehen.
IV. Abkühlphase des Absorptionsmaterials mittels des Wärmeträgerfluids 48a und Wärmeintegration bzw. Realisierung des TSA Prozesses durch Übertragung einer Abwärme 86a an eine andere Absorptionseinheit 22a, 22a', 22a", 22a"', die sich in einer Aufheizphase III befindet. Eine Restabwärme 88a wird an die Biogasanlage 98a, und zwar vorzugsweise an den Fermenter, abgegeben.
V. Befüllung/Bedruckung des Absorptionsmaterials mit Biogas 10a aus der Membraneinheit 26a.

Zur Kühlung ist eine Kühleinheit 28a in Form einer Absorptionskälteanlage vorgesehen, wobei eine Restwärme-Kopplung mit der Biogasanlage 98a vorgenommen wird (Fig. 6).

Wie zuvor ausgeführt, wird über die Ventile V1, V2, V9, V10, V11, V13 ein PSA Prozess bzw. ein Druckwechsel zwischen den Absorptionseinheiten 22a, 22a', 22a", 22a'" realisiert. Hierbei wird Druckenergie 90a von einer Absorptionseinheit 22a, 22a', 22a", 22a'" zu einer anderen Absorptionseinheit 22a, 22a', 22a", 22a'" übertragen, und zwar in der Weise, dass ein Druck zu Beginn der Absorptionsphase AP erhöht und ein Druck zu Beginn der Regenerationsphase RP reduziert wird. Im dargestellten Ausführungsbeispiel sind hierzu die Absorptionseinheit 22a und die Absorptionseinheit 22a" miteinander verschaltet und es sind die Absorptionseinheit 22a' und die Absorptionseinheit 22a'" miteinander verschaltet.

Am Ausgang der Anlage sind ein Strömungssensor S2 und ein CH₄-Sensor S3 angeordnet.

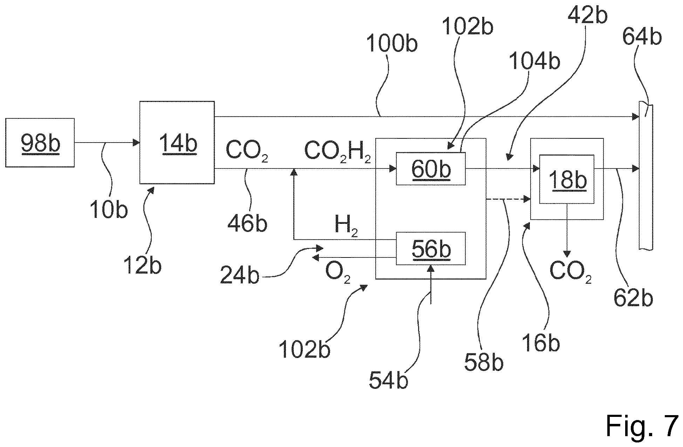

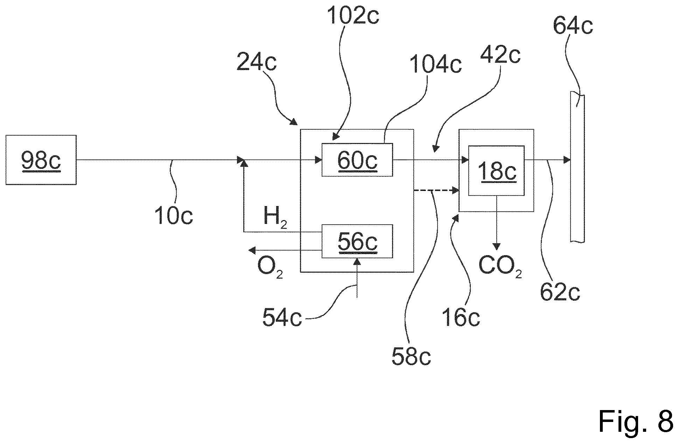

In den Figuren 7 und 8 sind weitere nicht erfindungsgemäße Ausführungsbeispiele gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung des ersten Ausführungsbeispiels verwiesen werden kann. Zur Unterscheidung der Ausführungsbeispiele sind den Bezugszeichen die Buchstaben a, b und c hinzugefügt. Bezüglich gleich bezeichneter Bauteile, insbesondere in Bezug auf Bauteile mit gleichen Bezugszeichen, kann grundsätzlich auch auf die Zeichnungen und/oder die Beschreibung des ersten Ausführungsbeispiels in den Figuren 1 bis 6 verwiesen werden.

In den Figuren 7 und 8 sind zwei weitere vereinfachte Prozessdarstellungen gezeigt, in denen ein Absorptionsprozess 18b, 18c in nicht näher dargestellten Absorptionseinheiten zur Gasaufbereitung eingesetzt wird, insbesondere zur Feinreinigung eines erzeugten Methans 62a, 62b einer Power-to-Gas Anlage 24b, 24c, wodurch vor der Einspeisung in ein Erdgasnetz 64b, 64c eine sehr hohe Reinheit des Methans 62b, 62c erzielt wird. Die Absorptionseinheiten in den Figuren 7 und 8 sind entsprechend den Absorptionseinheiten 22a, 22a', 22a", 22a'" des Ausführungsbeispiels in den Figuren 1 bis 6 ausgebildet. Der Prozess in Figur 7 zeigt dabei einen Prozess, indem die Gasaufbereitung in einer Biogasanlage 98b mit einem Membranprozess 14b durchgeführt wird und der Prozess in Figur 8 zeigt einen Prozess, indem die Gasaufbereitung in einer Biogasanlage 98c ohne Membranprozess ausgeführt ist.

Bei dem Prozess in Figur 7 wird ein von dem Membranprozess 14b abgetrenntes CO₂ bzw. Permeat 46b der Power-to-Gas Anlage 24b zugeführt und das Permeat 46b wird zusammen mit H₂ in einer Methanisierung 60b in einem Verfahrensschritt 102b in einem reaktiven Prozess 104b zu Methan und Wasser umgesetzt. Das Produktgas nach der Methanisierung 60b weist Reinheiten größer 90 Vol.% Methan auf. In einem nachgeschalteten Verfahrensschritt 16b wird in einem Absorptionsprozess 18b in den nicht näher dargestellten Absorptionseinheiten dann eine Gasfeinreinigung durchgeführt, wodurch das nicht umgesetzte CO₂ abgetrennt wird und das Produktgas zusätzlich getrocknet wird. Das abgetrennte CO₂ wird bevorzugt der Methanisierung 60b als reines CO₂ oder CO₂/H₂ Gasgemisch zurückgeführt. Es können mit dem Prozess nach Figur 7 sehr hohe Methanreinheiten im Gasstrom von bevorzugt größer 99,5 Vol.% Methan 62b erzielt werden. Im Anschluss wird das gewonnene Methan 62b in das Erdgasnetz 64b eingespeist. Ein sich aus dem Membranprozess 14b ergebendes hochreines Methan 100b, vorzugsweise mit einer Reinheit größer 99%, wird direkt in das Erdgasnetz 64b eingespeist. Alternativ könnte das Prozessgas des Membranprozesses 14b ebenfalls noch in einem Adsorptionsprozess und/oder Absorptionsprozess nachgereinigt werden.

Bei dem Prozess in Figur 8 wird anstelle einer vorgeschalteten CO₂ Abtrennung mittels eines Membranprozesses das im Biogas 10c enthaltene CO₂ in einem Verfahrensschritt 102c in einem reaktiven Prozess 104c durch eine katalytische Reaktion mit H₂ in einer Methanisierungseinheit 60c umgesetzt. Im Anschluss wird eine Gasfeinreinigung des Produktgases in einem nachgeschalteten Verfahrensschritt 16c in einem Absorptionsprozess 18c in Absorptionseinheiten in analoger Weise wie beim Prozess in Figur 7 durchgeführt. Bevor das Biogas 10c der Power-to-Gas Anlage 24c zugeführt wird, wird das Biogas 10c getrocknet und vorgereinigt, insbesondere entschwefelt.

## Patentansprüche

1. Verfahren zu einer Aufbereitung von Biogas (10a) einer Biogasanlage (98a), bei dem in einem Verfahrensschritt (12a) zu einer CO₂-Abtrennung aus dem Biogas (10a) ein Membranprozess (14a) in einer Membraneinheit (26a) durchgeführt wird und in einem weiteren, nachgeschalteten Verfahrensschritt (16a) ein Adsorptions- und/oder Absorptionsprozess (18a) in Absorptionseinheiten 22a, 22a', 22a", 22a'" zur Gasreinigung zur Erzielung einer Methanreinheit von größer als 99,5 Vol.% durchgeführt wird, wobei der Adsorptions- und/oder Absorptionsprozess (18a, 18b) als Nachreinigung durchgeführt wird, wobei in dem Adsorptions- und/oder Absorptionsprozess (18a) ein Festkörperadsorber und/oder ein Festkörperabsorber (20a) genutzt wird, und zwar ein Festamin, wobei in einem weiteren Verfahrensschritt (102a) ein reaktiver Prozess (104a), bei dem in einer Power-to-Gas Anlage (24a) in einem katalytisch aktivierten chemischen Prozess ein methanreiches Gasgemisch erzeugt wird, durchgeführt wird, wobei bei der Power-to-Gas Anlage (24a) elektrische Energie aus einer Anlage, wie insbesondere einer Windkraftanlage und/oder einer Solaranlage, verwendet wird, um mittels Elektrolyse Wasserstoff zu erzeugen, wobei in der Power-to-Gas Anlage (24a) erzeugter Wasserstoff während der Regeneration der Adsorptions- und/oder Absorptionseinheit (22a, 22a', 22a", 22a'") zugeführt wird und wobei die Adsorptions- und/oder Absorptionseinheit (22a, 22a', 22a", 22a'") eine Funktion einer CO₂-Speicherung und einer Gasvormischung für eine sich anschließende Methanisierung (60a) in der Power-to-Gas Anlage (24a) übernehmen, wobei der Wasserstoff während Regenerationsphasen der Absorptionseinheiten (22a, 22a', 22a", 22a'") in die Absorptionseinheiten (22a, 22a', 22a", 22a'") bei erhöhtem Druck eingeleitet wird und ein in den Absorptionseinheiten (22a, 22a', 22a", 22a'") befindliches Absorptionsmaterial unter der H₂-Atmosphäre thermisch regeneriert wird, wobei eine Abwärme (58a) der Power-to-Gas Anlage (24a), und zwar von der Elektrolyse (56a), die von einem Elektrolyseur durchgeführt wird, oder von einer Methanisierung (60a), zur Regeneration des Absorptionsmaterials verwendet wird, wobei ein frei werdendes CO₂/H₂ Gasgemisch dann unter Druck einer sich anschließenden Methanisierung (60a) in der Power-to-Gas Anlage (24a) zugeführt wird.

2. Verfahren nach Anspruch 1, **dadurch gekennzeichnet, dass** zumindest eine Adsorptions- und/oder Absorptionseinheit (22a, 22a', 22a", 22a'") in zumindest einem Verfahrensschritt (12a, 16a) gekühlt oder erwärmt wird.

3. Verfahren nach Anspruch 2, **dadurch gekennzeichnet, dass** Wärmeenergie von zumindest einer Adsorptions- und/oder Absorptionseinheit (22a, 22a', 22a", 22a'") zu zumindest einer weiteren Adsorptions- und/oder Absorptionseinheit (22a, 22a', 22a", 22a'") übertragen wird.

4. Verfahren nach einem der vorhergehenden Ansprüche, **dadurch gekennzeichnet, dass** Druckenergie (90a) von zumindest einer Adsorptions- und/oder Absorptionseinheit (22a, 22a', 22a", 22a'") zu zumindest einer weiteren Adsorptions- und/oder Absorptionseinheit (22a, 22a', 22a", 22a'") übertragen wird.

5. Vorrichtung zu einer Durchführung eines Verfahrens nach einem der vorhergehenden Ansprüche zu einer Aufbereitung von Biogas (10a) einer Biogasanlage (98a), mit einer Membraneinheit (26a), mit wenigstens einer ersten Adsorptions- und/oder Absorptionseinheit (22a, 22a', 22a", 22a'"), die ein Festkörperadsorber und/oder ein Festkörperabsorber (20a), und zwar ein Festamin, aufweist, und mit zumindest einem Anlagenanschluss (42a) an welchem eine reaktive Anlage, und zwar einer Power-to-Gas Anlage (24a) mit einem Elektrolyseur und mit einer Methanisierung (60a), derart angeschlossen ist, dass in der Power-to-Gas Anlage (24a) erzeugter Wasserstoff während der Regeneration der Adsorptions- und/oder Absorptionseinheit (22a, 22a', 22a", 22a'") zugeführt wird und frei werdendes CO₂/H₂ Gasgemisch unter Druck der Methanisierung (60a) in der Power-to-Gas Anlage (24a) zugeführt wird, wobei Mittel zur Nutzung einer freiwerdenden Reaktionswärme der Methanisierung (60a) oder Abwärme des Elektrolyseurs der Power-to-Gas Anlage (24a) bei der Adsorptions- und/oder Absorptionseinheit (22a, 22a', 22a", 22a'") bei einer Regeneration vorgesehen sind.

6. Vorrichtung nach Anspruch 5, **gekennzeichnet durch** zumindest eine Heiz- und/oder Kühleinheit (28a, 30a, 32a, 32a', 32a", 32a'").

7. Vorrichtung nach Anspruch 6, **dadurch gekennzeichnet, dass** die Heiz- und/oder Kühleinheit (28a, 30a, 32a, 32a', 32a", 32a'") zumindest einen Wärmetauscher umfasst.

8. Vorrichtung nach Anspruch 7, **dadurch gekennzeichnet, dass** in den Wärmetauscher wenigstens ein Adsorptions- und/oder Absorptionselement integriert ist.

9. Vorrichtung nach Anspruch 8, **dadurch gekennzeichnet, dass** der Wärmetauscher wenigstens ein Rohr (34a, 36a) umfasst, welches zumindest teilweise mit einem Adsorptions- und/oder Absorptionsmaterial gefüllt ist.

10. Vorrichtung nach Anspruch 9, **dadurch gekennzeichnet, dass** das Rohr (36a) zumindest eine erste und eine zweite Wandung (38a, 40a) aufweist und das Adsorptions- und/oder Absorptionsmaterial zumindest teilweise zwischen den Wandungen (38a, 40a) angeordnet ist.

11. Vorrichtung nach einem der Ansprüche 5 bis 10, **gekennzeichnet durch** zumindest eine zweite Adsorptions- und/oder Absorptionseinheit (22a, 22a', 22a", 22a'"), die mit der ersten Adsorptions- und/oder Absorptionseinheit (22a, 22a', 22a", 22a'") verschaltet ist.

## Claims

1. Method for a processing of biogas (10a) of a biogas plant (98a), in which in one method step (12a) a membrane process (14a) is executed in a membrane unit (26a) for a CO₂ separation from the biogas (10a), and in a further, downstream method step (16a) an adsorption and/or absorption process (18a) is executed in absorption units (22a, 22a', 22a", 22a'") for a gas purification to achieve a methane purity of more than 99.5 vol.%, wherein the adsorption and/or absorption process (18a, 18b) is executed as a subsequent purification, wherein a solid-matter adsorber and/or solid-matter absorber (20a), namely a solid amine, is used in the adsorption and/or absorption process (18a), wherein in a further method step (102a) a reactive process (104a) is executed in a power-to-gas plant (24a), in which a methane-rich gas mixture is generated in a catalytically activated chemical process, wherein electrical energy from a plant, like in particular a wind power plant and/or a solar plant, is used in the power-to-gas plant (24a) to generate hydrogen via electrolysis, wherein hydrogen generated in the power-to-gas plant (24a) is conveyed to the adsorption and/or absorption unit (22a, 22a', 22a", 22a'") during regeneration, and wherein the adsorption and/or absorption unit (22a, 22a', 22a", 22a'") takes on a function of CO₂ storage and gas premixing for a subsequent methanisation (60a) in the power-to-gas plant (24a), wherein during regeneration phases of the absorption units (22a, 22a', 22a", 22a'") the hydrogen is introduced into the absorption units (22a, 22a', 22a", 22a'") under elevated pressure and an absorption material located in the absorption units (22a, 22a', 22a", 22a'") is thermally regenerated under the H₂ atmosphere, wherein a waste heat (58a) of the power-to-gas plant (24a), from the electrolysis (56a) executed by an electrolyser or from a methanisation (60a), is used for a regeneration of the absorption material, wherein a released CO₂/H₂ gas mixture is then fed to a following methanisation (60a) in the power-to-gas plant (24a) under pressure.

2. Method according to claim 1, **characterised in that** at least one adsorption and/or absorption unit (22a, 22a', 22a", 22a'") is cooled or heated in at least one method step (12a, 16a).

3. Method according to claim 2, **characterised in that** heat energy is transferred from at least one adsorption and/or absorption unit (22a, 22a', 22a", 22a'") to at least one further adsorption and/or absorption unit (22a, 22a', 22a", 22a'").

4. Method according to one of the preceding claims, **characterised in that** a pressure energy (90a) is transferred from at least one adsorption and/or absorption unit (22a, 22a', 22a", 22a'") to at least one further adsorption and/or absorption unit (22a, 22a', 22a", 22a'").

5. Device for executing a method according to one of the preceding claims, for a processing of biogas (10a) of a biogas plant (98a), with a membrane unit (26a), with at least one first adsorption and/or absorption unit (22a, 22a', 22a", 22a'") comprising a solid-matter adsorber and/or solid-matter absorber (20a), namely a solid amine, and with at least one plant connection, in which a reactive plant, namely a power-to-gas plant (24a) with an electrolyser and with a methanisation (60a), is connected in such a way that hydrogen generated in the power-to-gas plant (24a) is conveyed to the adsorption and/or absorption unit (22a, 22a', 22a", 22a'") during regeneration and a released CO₂/H₂ gas mixture is conveyed to the methanisation (60a) in the power-to-gas plant (24a) under pressure, wherein means are provided for a utilisation of a released reaction heat of the methanisation (60a) or of a waste heat of the electrolyser of the power-to-gas plant (24a) in the adsorption and/or absorption unit (22a, 22a', 22a", 22a'") in a regeneration.

6. Device according to claim 5, **characterised by** at least one heating and/or cooling unit (28a, 30a, 32a, 32a', 32a", 32a'").

7. Device according to claim 6, **characterised in that** the heating and/or cooling unit (28a, 30a, 32a, 32a', 32a", 32a'") comprises at least one heat exchanger.

8. Device according to claim 7, **characterised in that** at least one adsorption and/or absorption element is integrated in the heat exchanger.

9. Device according to claim 8, **characterised in that** the heat exchanger comprises at least one tube (34a, 36a), which is at least partially filled with an adsorption and/or absorption material.

10. Device according to claim 9, **characterised in that** the tube (36a) comprises at least one first and a second wall (38a, 40a) and the adsorption and/or absorption material is arranged at least partially between the walls (38a, 40a).

11. Device according to one of claims 5 to 10, **characterised by** at least one second adsorption and/or absorption unit (22a, 22a', 22a", 22a'"), which is interconnected with the first adsorption and/or absorption unit (22a, 22a', 22a", 22a'").

## Revendications

1. Procédé pour un traitement de biogaz (10a) d'une installation biogaz (98a), dans lequel, dans une étape de procédé (12a), un procès de membrane (14a) est exécuté dans une unité membrane (26a) pour une séparation de CO2 du biogaz (10a) et, dans une autre étape de procédé en aval (16a), un procès d'adsorption et/ou absorption (18a) est exécuté dans des unités d'absorption (22a, 22a', 22a", 22a"') pour une purification de gaz pour le bût d'achever une pureté de méthane supérieure à 99,5 % vol, le procès d'adsorption et/ou absorption (18a, 18b) étant exécuté comme purification de suivi, un adsorbant solide et/ou un absorbant solide (20a) étant utilisé dans le procès d'adsorption et/ou absorption (18a), notamment une amine solide, un procès réactif (104a) étant exécuté dans une autre étape de procédé (102a), dans lequel procès réactif (104a) une mixture de gaz riche en méthane est produite en un procès chimique activé catalytiquement dans une installation power-to-gas [conversion d'électricité en gaz] (24a), de l'énergie électrique d'une installation, comme particulièrement une installation éolienne et/ou une installation solaire, étant utilisée dans l'installation power-to-gas (24a) pour produire de l'hydrogène par électrolyse, l'hydrogène produit dans l'installation power-to-gas (24a) étant alimenté à l'unité d'adsorption et/ou d'absorption (22a, 22a', 22a", 22a"') pendant la régénération et l'unité d'adsorption et/ou d'absorption (22a, 22a', 22a", 22a"') prenant la fonction d'un stockage de CO2 et d'un pré-mixage de gaz pour une méthanisation (60a) suivante dans l'installation power-to-gas (24a), l'hydrogène étant introduit dans les unités d'adsorption et/ou d'absorption (22a, 22a', 22a", 22a'"), sous pression élevée, pendant les phases régénératives des unités d'adsorption et/ou d'absorption (22a, 22a', 22a", 22a"') et un matériau d'absorption situé dans les unités d'adsorption et/ou d'absorption (22a, 22a', 22a", 22a"') étant régénéré thermiquement sous l'atmosphère à hydrogène,
une chaleur d'échappement (58a) de l'installation power-to-gas (24a), à savoir de l'électrolyse (56a) réalisée par un électrolyseur, ou d'une méthanisation (60a), étant utilisée pour une régénération du matériau d'absorption,
une mixture de gaz CO2/H2 libérée étant puis alimentée, sous pression, à une méthanisation (60a) suivante dans l'installation power-to-gas (24a).

2. Procédé selon la revendication 1,
**caractérisé en ce qu'**au moins une unité d'adsorption et/ou d'absorption (22a, 22a', 22a", 22a"') est refroidi ou chauffée dans au moins une étape de procédé (12a, 16a).

3. Procédé selon la revendication 2,
**caractérisé en ce qu'**une énergie thermique est transférée d'au moins une unité d'adsorption et/ou d'absorption (22a, 22a', 22a", 22a"') à au moins une autre unité d'adsorption et/ou d'absorption (22a, 22a', 22a", 22a'").

4. Procédé selon l'une quelconque des revendications précédentes,
**caractérisé en ce qu'**une énergie de pression (90a) est transférée d'au moins une unité d'adsorption et/ou d'absorption (22a, 22a', 22a", 22a"') à au moins une autre unité d'adsorption et/ou d'absorption (22a, 22a', 22a", 22a'").

5. Dispositif pour une réalisation d'un procédé selon l'une quelconque des revendications précédentes pour un traitement de biogaz (10a) d'une installation biogaz (98a), avec une unité membrane (26a), avec au moins une première unité d'adsorption et/ou d'absorption (22a, 22a', 22a", 22a"') comprenant un adsorbant solide et/ou un absorbant solide (20a), notamment une amine solide, et avec au moins un raccordement d'installation (42a), auquel une installation réactive, notamment une installation power-to-gas (24a) avec un électrolyseur et avec une méthanisation (60a), est raccordée de telle manière que de l'hydrogène produit dans l'installation power-to-gas (24a) est alimenté, pendant la régénération, à l'unité d'adsorption et/ou d'absorption (22a, 22a', 22a", 22a"') et une mixture de gaz CO2/H2 est alimentée, sous pression, à la méthanisation (60a) dans l'installation power-to-gas (24a),
des moyens pour une utilisation d'une chaleur de réaction de la méthanisation (60a) ou d'une chaleur d'échappement de l'électrolyseur de l'installation power-to-gas (24a) étant prévus dans l'unité d'adsorption et/ou d'absorption (22a, 22a', 22a", 22a"') dans une régénération.

6. Dispositif selon la revendication 5, **caractérisé par** au moins une unité de chauffage et/ou de refroidissement (28a, 30a, 32a, 32a', 32a", 32a'").

7. Dispositif selon la revendication 6, **caractérisé en ce que** l'unité de chauffage et/ou de refroidissement (28a, 30a, 32a, 32a', 32a", 32a"') comporte au moins un échangeur de chaleur.

8. Dispositif selon la revendication 7, **caractérisé en ce qu'**au moins un élément d'adsorption et/ou d'absorption est intégré dans l'échangeur de chaleur.

9. Dispositif selon la revendication 8, **caractérisé en ce que** l'échangeur de chaleur comporte au moins un tuyau (34a, 36a), qui est au moins partiellement rempli d'un matériau d'adsorption et/ou d'absorption.

10. Dispositif selon la revendication 9, **caractérisé en ce que** le tuyau (36a) comporte au moins une première paroi et une deuxième paroi (38a, 40a) et que le matériau d'adsorption et/ou absorption est disposé au moins partiellement entre les parois (38a, 40a).

11. Dispositif selon l'une quelconque des revendications 5 à 10, **caractérisé par** au moins une deuxième unité d'adsorption et/ou d'absorption (22a, 22a', 22a", 22a"') interconnectée avec la première unité d'adsorption et/ou d'absorption (22a, 22a', 22a", 22a'").
